# EUROPEAN PATENT SPECIFICATION

(11) **EP 1 589 967 B1**
(45) Date of publication and mention of the grant of the patent: **09.05.2007**
(21) Application number: 04705459.8
(22) Date of filing: 27.01.2004
(51) Int. Cl.: A61K 31/428, C07D 277/68, A61P 35/00

(54) **BENZTHIAZOLE-3 OXIDES USEFUL FOR THE TREATMENT OF PROLIFERATIVE DISORDERS**
BENZTHIAZOL-3 OXIDE ZUR BEHANDLUNG VON PROLIFERATIVEN STÖRUNGEN
OXYDES DE BENZOTHIAZOLE-3 UTILES POUR LE TRAITEMENT DE TROUBLES PROLIFERATIFS

(30) Priority: 30.01.2003 GB 0302220
(43) Date of publication of application: 02.11.2005
(73) Proprietor: Cyclacel Limited, London SW1Y 4RB (GB)
(72) Inventor: MCINNES, Campbell, Longforgan, Dundee DD2 5JE (GB); MEADES, Christopher, Whitehazel Park, Dundee DD4 0TE (GB); MEZNA, Mokdad, Dundee DD2 5JA (GB); FISCHER, Peter, Angus DD11 2EN, Scotland (GB)
(74) Representative: Clyde-Watson, Zöe
(86) International application number: PCT/GB2004/000327
(87) International publication number: WO 2004/067000

(56) References cited:
- EP-A- 0 334 138
- WO-A-00/04901
- DE-A- 2 013 434
- US-A- 3 691 186

## Description

The present invention relates to the use of heterocyclic N-oxides as therapeutic agents in the treatment of proliferative disorders. More specifically, but not exclusively, the invention relates to the use of heterocyclic N-oxides that are capable of inhibiting polo-like kinases (PLKs).

### BACKGROUND TO THE INVENTION

The therapeutic potential of pharmacological PLK inhibition has been noted in the literature (Kraker et al., in: Annual Reports in Medicinal Chemistry; Academic Press: San Diego, CA, 1999; pp 247). As yet though, there have been very few reports of small-molecule PLK inhibitors that may be useful as drugs.

To date, the only characterized biochemical PLK1 inhibitor is scytonemin, a symmetric indolic marine natural product (Stevenson et al., J. Pharmacol. Exp. Ther., 2002, 303, 858; Jacobs et al; WO 2001/062900; University of California). Scytonemin inhibits phosphorylation of CDC25C by recombinant PLK1 with an IC₅₀ value of about 2 µM (at an ATP concentration of 10 µM). Inhibition is apparently reversible and the mechanism with respect to ATP is of mixed-competitive mode. Similar potency against other Ser/Thr and Thr/Tyr cell-cycle kinases, including MYT1, CHK1, CDK1/cyclin B, and PKC, was observed. Scytonemin was shown to have pronounced anti-proliferative effects on various human cell lines *in vitro.*

Certain 2-benzthiazolyl urea derivatives have been disclosed in the art as protein kinase inhibitors (Cusack et al., WO 2001/057008; BASF). However, there was no evidence to indicate that the 2-benzthiazolyl urea derivatives were capable of inhibiting PLKs. There are also reports in the literature of benzthiazole-3-oxides having antibacterial and antifungal properties (see for example, Wagner et al., Ger. Offen. 2136923, 36 pp.; Bayer A.-G.: Germany, 1973). Again though, there was no indication that the biological activity of these benzthiazole-3-oxides was in any way associated with protein kinase inhibition in general, nor was there any reference to PLK inhibition.

DE 2013434 (BAYER AG) discloses benzthiazole-3-oxides according to a general formula II as medicaments, namely as concrement inhibitors and for the treatment of urinary calculus.

WO 00/04901 (UNIV JEFFERSON) discloses a benzthiazole-3-oxide, namely the compound HA02, as an active agent to induce apoptosis and for treating cancer and autoimmune disorders.

The present invention seeks to provide heterocyclic N-oxide compounds that have therapeutic applications in the treatment of a range of proliferative disorders.

### STATEMENT OF INVENTION

A first aspect of the invention relates to the use of a compound of formula I, or a pharmaceutically acceptable salt thereof, 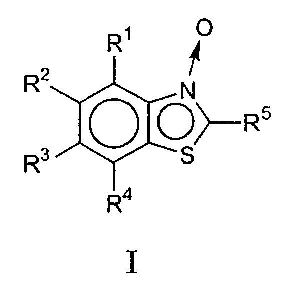 wherein
R¹, R², R³, and R⁴ are each independently H, NO₂, CF₃, SCF₃, CN, halo, OH, OR⁶, NH₂, NHR⁶, NR⁶R⁷, N⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶, SO₂NH₂, SO₂NHR⁶, SO₂NR⁶R⁷, alkyl, cycloalkyl, cycloheteroakyl, aryl, or heteroaryl, with the proviso that at least one of R¹, R², R³, and R⁴ is other than H; or R¹ and R², R² and R³, or R³ and R⁴, may together form part of a fused or unfused saturated or unsaturated ring system, optionally containing up to two heteroatoms selected from N, O, and S;
R⁵ is OH, OR⁹, CN, CONH₂, CONHNH₂, CONHOH, CONHR⁹, CONR⁹R¹⁰, NH₂, NHR⁹, or NR⁹R¹⁰;
each R⁶, R⁷ and R⁸ is independently hydrocarbyl, or two of R⁶, R⁷ and R⁸ together form part of a saturated or unsaturated ring system, optionally containing up to two heteroatoms selected from N, O, and S;
each R⁹ and R¹⁰ is independently hydrocarbyl, or R⁹ and R¹⁰ together form part of a saturated or unsaturated ring system, optionally containing up to two heteroatoms selected from N, O, and S;
in the preparation of a medicament for treating a proliferative disorder.

Further aspects of the invention relate to selected compounds of formula I, and pharmaceutical compositions comprising said compounds admixed with a pharmaceutically acceptable carrier, diluent or excipient.

A further aspect of the invention relates to the use of a compound of formula I, or a pharmaceutically acceptable salt thereof, as defined above, in an assay for identifying further candidate compounds capable of inhibiting PLK.

### DETAILED DESCRIPTION

As used herein, the term "hydrocarbyl" refers to a saturated or unsaturated, straight-chain, branched, or cyclic group comprising at least C and H that may optionally comprise one or more other suitable substituents. Examples of such substituents may include halo, hydroxy, CF₃, CN, amino and nitro. If the hydrocarbyl group comprises more than one C then those carbons need not necessarily be linked to each other. For example, at least two of the carbons may be linked via a suitable element or group. Thus, the hydrocarbyl group may contain heteroatoms. Suitable heteroatoms will be apparent to those skilled in the art and include, for instance, sulphur, nitrogen, oxygen, phosphorus and silicon. Preferably, the hydrocarbyl group is an aryl or alkyl group.

As used herein the term "alkyl" includes both straight chain and branched alkyl groups. The alkyl group may be substituted (mono- or poly-) or unsubstituted. Suitable substituents include, for example, OH, COOH, halo-, alkoxy-, nitro-, CN, CF₃ or a cyclic group. Preferably, the alkyl group is a C₁₋₂₀ alkyl group, more preferably a C₁₋₁₅, more preferably still a C₁₋₁₂ alkyl group, more preferably still, a C₁₋₆ alkyl group, more preferably a C₁₋₃ alkyl group. Particularly preferred alkyl groups include, for example, methyl, ethyl, propyl, isopropyl, butyl, isobutyl, tert-butyl, pentyl and hexyl.

As used herein, the term "heteroalkyl" includes an alkyl group as defined above which comprises one or more heteroatoms.

As used herein, the term "cycloalkyl" refers to a cyclic alkyl group which may be substituted (mono- or poly-) or unsubstituted. Suitable substituents include, for example, OH, COOH, halo-, alkoxy-, nitro-, CN, CF₃ or a cyclic group.

Likewise, the term "cycloheteroalkyl" refers to a cyclic heteroalkyl group which may be substituted (mono- or poly-) or unsubstituted. Suitable substituents include, for example, OH, COOH, halo-, alkoxy-, nitro-, CN, CF₃ or a cyclic group.

As used herein, the term "aryl" refers to a C₆₋₁₀ aromatic, substituted (mono- or poly-) or unsubstituted group, and includes, for example, phenyl, naphthyl etc. Again, suitable substituents include, for example, OH, COOH, halo-, alkoxy-, nitro-, CN, CF₃ or a cyclic group.

As used herein, the term "heteroaryl" refers to a C₄₋₁₀ aromatic, substituted (mono- or poly-) or unsubstituted group, which comprises one or more heteroatoms. Preferred heteroaryl groups include pyrrole, pyrazole, pyrimidine, pyrazine, pyridine, quinoline, thiophene and furan. Again, suitable substituents include, for example, OH, COOH, halo-, alkoxy-, nitro-, CN, CF₃ or a cyclic group.

As used herein the phrase "preparation of a medicament" includes the use of a compound of formula I directly as the medicament in addition to its use in a screening programme for further antiproliferative agents or in any stage of the manufacture of such a medicament.

In one preferred embodiment of the invention:
R¹, R², R³, and R⁴ are each independently H, NO₂, CF₃, SCF₃, CN, halo, OH, OR⁶, NH₂, NHR⁶, NR⁶R⁷, N⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶, SO₂NH₂, SO₂NHR⁶, SO₂NR⁶R⁷, alkyl, cycloalkyl, cycloheteroalkyl, aryl, or heteroaryl, with the proviso that at least one of R¹, R², R³, and R⁴ is other than H; or R¹ and R², R² and R³, or R³ and R⁴, may together form part of a fused or unfused saturated or unsaturated ring system, optionally containing up to two heteroatoms selected from N, O, and S;
R⁵ is OH, OR⁹, CN, CONH₂, CONHNH₂, CONHOH, CONHR⁹, CONR⁹R¹⁰, NH₂, NHR⁹, or NR⁹R¹⁰;
each R⁶, R⁷, R⁸, R⁹ and R¹⁰ is independently hydrocarbyl, or two of R⁶, R⁷, and R⁸ may together form part of a saturated or unsaturated ring system.

In one preferred embodiment, R¹, R², R³, and R⁴ are each independently H, NO₂, CF₃, SCF₃, CN, halo, OH, OR⁶, NH₂, NHR⁶, NR⁶R⁷, N⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶, SO₂NH₂, SO₂NHR⁶, SO₂NR⁶R⁷, alkyl, cycloalkyl, cycloheteroalkyl, aryl, or heteroaryl.

In one preferred embodiment, each R⁶, R⁷ and R⁸ is independently hydrocarbyl, or two of R ⁶, R⁷ and R⁸ together form part of a saturated ring system, optionally containing up to two heteroatoms selected from N, O, and S.

In one preferred embodiment, each R⁹ and R¹⁰ is independently hydrocarbyl, or R⁹ and R¹⁰ together form part of a saturated ring system, optionally containing up to two heteroatoms selected from N, O, and S.

In one particularly preferred embodiment, each R⁶, R⁷ and R⁸ is independently hydrocarbyl, or two of R⁶, R⁷ and R⁸ together form part of a saturated ring system.

In another particularly preferred embodiment, each R⁹ and R¹⁰ is independently hydrocarbyl, or R⁹ and R¹⁰ together form part of a saturated ring system.

In the case where each R⁶, R⁷, R⁸, R⁹ and R¹⁰ is independently hydrocarbyl, preferably the hydrocarbyl group is an alkyl or aryl group, more preferably an alkyl group.

More preferably, R⁵ is OH, CN, CONH₂, CONHNH₂, CONHOH or CONR⁹R¹⁰.

Preferably, where R⁵ is CONR⁹R¹⁰, R⁹ and R¹⁰ together form part of a saturated ring system, optionally containing up to two heteroatoms selected from N, O, and S. Even more preferably, R⁹ and R¹⁰ together form part of a saturated ring system. More preferably still, R⁹ and R¹⁰ together form part of a pyrrolidine ring, i.e. R⁵ is CO-pyrrolidine.

In one particularly preferred embodiment, R⁵ is selected from CONH₂, CO-pyrrolidine, CONHNH₂, CONHOH, CN and OH.

In a more preferred embodiment of the invention, R¹, R², R³, and R⁴ are each independently H, NO₂, CF₃, SCF₃ or halo.

In one particularly preferred embodiment, R² is selected from CF₃, F, SCF₃ and H.

In another particularly preferred embodiment, R³ and R¹ are both H.

In another particularly preferred embodiment, R⁴ is NO₂.

In one especially preferred embodiment, the compound of formula I is selected from the following:
7-Nitro-3-oxy-5-trifluoromethyl-benzothiazole-2-carboxylic acid amide;
(7-Nitro-3-oxy-5-trifluoromethyl-benzothiazol-2-yl)-pyrrolidin-1-yl-methanone;
7-Nitro-3-oxy-5-trifluoromethyl-benzothiazole-2-carboxylic acid hydrazide;
5-Cyano-7-nitro-3-oxy-benzothiazole-2-carboxylic acid amide;
7-Nitro-3-oxy-5-trifluoromethyl-benzothiazole-2-carboxylic acid hydroxyamide;
5-Fluoro-7-nitro-3-oxy-benzothiazole-2-carboxylic acid amide;
5-Fluoro-7-nitro-3-oxy-benzothiazole-2-carboxylic acid hydroxyamide;
7-Nitro-3-oxy-5-trifluoromethyl-benzothiazole-2-carbonitrile;
7-Nitro-3-oxy-5-trifluoromethyl-benzothiazol-2-ol; and
7-Nitro-3-oxy-5-trifluoromethylsulfanyl-benzothiazole-2-carboxylic acid amide.

Even more preferably, the compound of formula I is capable of inhibiting PLK. Preferably, the compound is capable of inhibiting PLK1 as measured by an appropriate kinase assay. Details of a suitable assay may be found in the accompanying examples section. Preferably, the compound of formula I exhibits an IC₅₀ value in a PLK1 kinase assay of less than 100 µM, more preferably less than 50 µM, more preferably still, less than 25 µM. More preferably, the compound of formula I is selected from the following:
7-Nitro-3-oxy-5-trifluoromethyl-benzothiazole-2-carboxylic acid amide;
5-Cyano-7-nitro-3-oxy-benzothiazole-2-carboxylic acid amide;
7-Nitro-3-oxy-5-trifluoromethyl-benzothiazole-2-carbonitrile; and
7-Nitro-3-oxy-5-trifluoromethylsulfanyl-benzothiazole-2-carboxylic acid amide.

More preferably still, the the compound of formula I exhibits an IC₅₀ value in a PLK1 kinase assay of less than 10 µM, more preferably less than 5 µM. In a highly preferred embodiment, the compound of formula I is selected from:
7-Nitro-3-oxy-5-trifluoromethyl-benzothiazole-2-carboxylic acid amide; and
7-Nitro-3-oxy-5-trifluoromethylsulfanyl-benzothiazole-2-carboxylic acid amide.

More preferably still, the compound of formula I exhibits an IC₅₀ value in a PLK1 kinase assay of less than 1 µM. In one highly preferred embodiment, the compound pf formula I is nitro-3-oxy-5-trifluoromethylsulfanyl-benzothiazole-2-carboxylic acid amide.

Even more preferably, the compound of formula I is capable of exhibiting an anti-proliferative effect as measured by an appropriate assay. Details of a suitable assay may be found in the accompanying examples section. Preferably, the compound of formula I exhibits an IC₅₀ value in an MTT assay using A549, HELA, MCF-7 or U2OS cell lines of less than 100 µM, more preferably less than 50 µM, more preferably still, less than 25 µM, even more preferably, less than 10 µM. Preferably, the compound is selected from the following:
(7-Nitro-3-oxy-5-trifluoromethyl-benzothiazol-2-yl)-pyrrolidin-1-yl-methanone;
5-Cyano-7-nitro-3-oxy-benzothiazole-2-carboxylic acid amide;
5-Fluoro-7-nitro-3-oxy-lienzothiazole-2-carboxylic acid amide;
5-Fluoro-7-nitro-3-oxy-benzothiazole-2-carboxylic acid hydroxyamide;
7-Nitro-3-oxy-5-trifluoromethyl-benzothiazole-2-carbonitrile; and
7-Nitro-3-oxy-5-trifluoromethylsulfanyl-benzothiazole-2-carboxylic acid amide.

### THERAPEUTIC APPLICATIONS

The compounds of formula I have been found to possess anti-proliferative activity and are therefore believed to be of use in the treatment of proliferative disorders such as cancers, leukaemias and other disorders associated with uncontrolled cellular proliferation such as psoriasis and restenosis. As defined herein, an anti-proliferative effect within the scope of the present invention may be demonstrated by the ability to inhibit cell proliferation in an *in vitro* whole cell assay, for example using any of the cell lines A549, HELA, MCF-7 or U2OS. These assays, including methods for their performance, are described in more detail in the accompanying Examples. Using such assays it may be determined whether a compound is anti-proliferative in the context of the present invention.

One aspect of the present invention therefore relates to the use of one or more compounds of formula I in the preparation of a medicament for the treatment of proliferative disorders. Preferably, the proliferative disorder is a cancer or leukaemia. The term proliferative disorder is used herein in a broad sense to include any disorder that requires control of the cell cycle, for example cardiovascular disorders such as restenosis and cardiomyopathy, auto-immune disorders such as glomerulonephritis and rheumatoid arthritis, dermatological disorders such as psoriasis, antiinflammatory, anti-fungal, antiparasitic disorders such as malaria, emphysema and alopecia. In these disorders, the compounds of the present invention may induce apoptosis or maintain stasis within the desired-cells as required.

In one preferred embodiment of the invention, the proliferative disorder is cancer.

In another preferred embodiment, the proliferative disorder is glomerulonephritis.

In yet another preferred embodiment, the proliferative disorder is rheumatoid arthritis.

In another preferred embodiment, the proliferative disorder is psoriasis.

In another preferred embodiment, the proliferative disorder is a chronic obstructive pulmonary disorder.

The compounds of the invention may inhibit any of the steps or stages in the cell cycle, for example, formation of the nuclear envelope, exit from the quiescent phase of the cell cycle (G0), G1 progression, chromosome decondensation, nuclear envelope breakdown, START, initiation of DNA replication, progression of DNA replication, termination of DNA replication, centrosome duplication, G2 progression, activation of mitotic or meiotic functions, chromosome condensation, centrosome separation, microtubule nucleation, spindle formation and function, interactions with microtubule motor proteins, chromatid separation and segregation, inactivation of mitotic functions, formation of contractile ring, and cytokinesis functions. In particular, the compounds of the invention may influence certain gene functions such as chromatin binding, formation of replication complexes, replication licensing, phosphorylation or other secondary modification activity, proteolytic degradation, microtubule binding, actin binding, septin binding, microtubule organising centre nucleation activity and binding to components of cell cycle signalling pathways.

In one embodiment of the invention, the compound of formula I is administered in an amount sufficient to inhibit at least one PLK enzyme.

The polo-like kinases (PLKs) constitute a family of serine/threonine protein kinases. Mitotic *Drosophila melanogaster* mutants at the *polo* locus display spindle abnormalities (Sunkel et al., J. Cell Sci., 1988, 89, 25) and *polo* was found to encode a mitotic kinase (Llamazares et al., Genes Dev., 1991, 5, 2153). In humans, there exist three closely related PLKs (Glover et al., Genes Dev., 1998, 12, 3777). They contain a highly homologous amino-terminal catalytic kinase domain and their carboxyl termini contain two or three conserved regions, the polo boxes. The function of the polo boxes remains incompletely understood but they are implicated in the targeting of PLKs to subcellular compartments (Lee et al., Proc. Natl. Acad. Sci. USA, 1998, 95, 9301; Leung et al., Nat. Struct. Biol., 2002, 9, 719), mediation of interactions with other proteins (Kauselmann et al., EMBO J., 1999, 18, 5528), or may constitute part of an autoregulatory domain (Nigg, Curr. Opin. Cell Biol., 1998, 10, 776). Furthermore, the polo box-dependent PLK1 activity is required for proper metaphase/anaphase transition and cytokinesis (Yuan et al., Cancer Res., 2002, 62, 4186; Seong et al., J. Biol. Chem., 2002, 277, 32282).

Studies have shown that human PLKs regulate some fundamental aspects of mitosis (Lane et al., J. Cell. Biol., 1996, 135, 1701; Cogswell et al., Cell Growth Differ., 2000, 11, 615). In particular, PLK1 activity is believed to be necessary for the functional maturation of centrosomes in late G2/early prophase and subsequent establishment of a bipolar spindle. Depletion of cellular PLK1 through the small interfering RNA (siRNA) technique has also confirmed that this protein is required for multiple mitotic processes and completion of cytokinesis (Liu et al., Proc. Natl. Acad. Sci. USA, 2002, 99, 8672).

In a more preferred embodiment of the invention, the compound of formula I is administered in an amount sufficient to inhibit PLK1.

Of the three human PLKs, PLK1 is the best characterized; it regulates a number of cell division cycle effects, including the onset of mitosis (Toyoshima-Morimoto et al., Nature, 2001, 410, 215; Roshak et al., Cell. Signalling, 2000, 12, 405), DNA-damage checkpoint activation (Smits et al., Nat. Cell Biol., 2000, 2, 672; van Vugt et al., J. Biol. Chem., 2001, 276, 41656), regulation of the anaphase promoting complex (Sumara et al., Mol. Cell, 2002, 9, 515; Golan et al., J. Biol. Chem., 2002, 277, 15552; Kotani et al., Mol. Cell, 1998, 1, 371), phosphorylation of the proteasome (Feng et al., Cell Growth Differ., 2001, 12, 29), and centrosome duplication and maturation (Dai et al., Oncogene, 2002, 21, 6195).

Specifically, initiation of mitosis requires activation of M-phase promoting factor (MPF), the complex between the cyclin dependent kinase CDK1 and B-type cyclins (Nurse, Nature, 1990, 344, 503). The latter accumulate during the S and G2 phases of the cell cycle and promote the inhibitory phosphorylation of the MPF complex by WEE1, MIK1, and MYT1 kinases. At the end of the G2 phase, corresponding dephosphorylation by the dual-specificity phosphatase CDC25C triggers the activation of MPF (Nigg, Nat. Rev. Mol. Cell Biol., 2001, 2, 21). In interphase, cyclin B localizes to the cytoplasm (Hagting et al., EMBO J., 1998, 17, 4127), it then becomes phosphorylated during prophase and this event causes nuclear translocation (Hagting et al., Curr. Biol., 1999, 9, 680; Yang et al., J. Biol. Chem., 2001, 276, 3604). The nuclear accumulation of active MPF during prophase is thought to be important for initiating M-phase events (Takizawa et al., Curr. Opin. Cell Biol., 2000, 12, 658). However, nuclear MPF is kept inactive by WEE1 unless counteracted by CDC25C. The phosphatase CDC25C itself, localized to the cytoplasm during interphase, accumulates in the nucleus in prophase (Seki et al., Mol. Biol. Cell, 1992, 3, 1373; Heald et al., Cell, 1993, 74, 463; Dalal et al., Mol. Cell. Biol., 1999, 19, 4465). The nuclear entry of both cyclin B (Toyoshima-Morimoto et al., Nature, 2001, 410, 215) and CDC25C (Toyoshima-Morimoto et al., EMBO Rep., 2002, 3, 341) are promoted through phosphorylation by PLK1 (Roshak et al., Cell. Signalling, 2000, 12, 405). This kinase is an important regulator of M-phase initiation.

In one particularly preferred embodiment, the compounds of formula I are ATP-antagonistic inhibitors of PLK1.

In the present context ATP antagonism refers to the ability of an inhibitor compound to diminish or prevent PLK catalytic activity, i.e. phosphotransfer from ATP to a macromolecular PLK substrate, by virtue of reversibly or irreversibly binding at the enzyme's active site in such a manner as to impair or abolish ATP binding.

In another preferred embodiment, the compound of formula I is administered in an amount sufficient to inhibit PLK2 and/or PLK3.

Mammalian PLK2 (also known as SNK) and PLK3 (also known as PRK and FNK) were originally shown to be immediate early gene products. PLK3 kinase activity appears to peak during late S and G2 phase. It is also activated during DNA damage checkpoint activation and severe oxidative stress. PLK3 also plays an important role in the regulation of microtubule dynamics and centrosome function in the cell and deregulated. PLK3 expression results in cell cycle arrest and apoptosis (Wang et al., Mol. Cell. Biol., 2002, 22, 3450). PLK2 is the least well understood homologue of the three PLKs. Both PLK2 and PLK3 may have additional important post-mitotic functions (Kauselmann et al., EMBO J., 1999,18, 5528).

### PHARMACEUTICAL COMPOSITIONS

In one preferred embodiment of the invention, the compound of formula I is administered in combination with a pharmaceutically acceptable excipient, diluent or carrier.

Even though the compounds of the present invention (including their pharmaceutically acceptable salts, esters and pharmaceutically acceptable solvates) can be administered alone, they will generally be administered in admixture with a pharmaceutical carrier, excipient or diluent, particularly for human therapy. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2^{nd} Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s).

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

### SALTS/ESTERS

The compounds of formula I can be present as salts or esters, in particular pharmaceutically acceptable salts or esters.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

### ENANTIOMERS/TAUTOMERS

In all aspects of the present invention previously discussed, the invention includes, where appropriate all enantiomers and tautomers of compounds of formula I. The man skilled in the art will recognise compounds that possess an optical properties (one or more chiral carbon atoms) or tautomeric characteristics. The corresponding enantiomers and/or tautomers may be isolated/prepared by methods known in the art.

### STEREO AND GEOMETRIC ISOMERS

Some of the specific compounds of formula I may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the compound or a pharmaceutically acceptable salt thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. Isotopic variations of the agent of the present invention and pharmaceutically acceptable salts thereof of this invention can generally be prepared by conventional procedures using appropriate isotopic variations of suitable reagents.

### SOLVATES

The present invention also includes the use of solvate forms of the compounds of the present invention. The terms used in the claims encompass these forms.

### POLYMORPHS

The invention furthermore relates to the compounds of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### PRODRUGS

The invention further includes the compounds of the present invention in prodrug form. Such prodrugs are generally compounds of formula I wherein one or more appropriate groups have been modified such that the modification may be reversed upon administration to a human or mammalian subject. Such reversion is usually performed by an enzyme naturally present in such subject, though it is possible for a second agent to be administered together with such a prodrug in order to perform the reversion in vivo. Examples of such modifications include ester (for example, any of those described above), wherein the reversion may be carried out be an esterase etc. Other such systems will be well known to those skilled in the art.

### ADMINISTRATION

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules. Preferably, these compositions contain from 1 to 250 mg and more preferably from 10-100 mg, of active ingredient per dose.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10 - 1000 mg, preferably between 10 - 250 mg, of active ingredient per dose.

Compositions may be formulated in unit dosage form, i.e., in the form of discrete portions containing a unit dose, or a multiple or sub-unit of a unit dose.

### DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

Depending upon the need, the agent may be administered at a dose of from 0.01 to 30 mg/kg body weight, such as from 0.1 to 10 mg/kg, more preferably from 0.1 to 1 mg/kg body weight.

In an exemplary embodiment, one or more doses of 10 to 150 mg/day will be administered to the patient for the treatment of malignancy.

### COMBINATIONS

In a particularly preferred embodiment, the one or more compounds of formula I are administered in combination with one or more other antiproliferative agents, for example, existing anticancer drugs available on the market. In such cases, the compounds of the invention may be administered consecutively, simultaneously or sequentially with the one or more other anticancer agents.

Anticancer drugs in general are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance in early tumor cells which would have been otherwise responsive to initial chemotherapy with a single agent. An example of the use of biochemical interactions in selecting drug combinations is demonstrated by the administration of leucovorin to increase the binding of an active intracellular metabolite of 5-fluorouracil to its target, thymidylate synthase, thus increasing its cytotoxic effects.

Numerous combinations are used in current treatments of cancer and leukemia. A more extensive review of medical practices may be found in "Oncologic Therapies" edited by E. E. Vokes and H. M. Golomb, published by Springer.

Beneficial combinations may be suggested by studying the growth inhibitory activity of the test compounds with agents known or suspected of being valuable in the treatment of a particular cancer initially or cell lines derived from that cancer. This procedure can also be used to determine the order of administration of the agents, i.e. before, simultaneously, or after delivery. Such scheduling may be a feature of all the cycle acting agents identified herein.

### COMPOUNDS

The present invention also relates to compounds of formula I.

More specifically, the invention relates to compounds of formula I selected from the following:
7-Nitro-3-oxy-5-trifluoromethyl-benzothiazol-2-yl)-pyrrolidin-1-yl-methanone;
5-Fluoro-7-nitro-3-oxy-benzothiazole-2-carboxylic acid amide;
5-Fluoro-7-nitro-3-oxy-benzothiazole-2-carboxylic acid hydroxyamide; and
7-Nitro-3-oxy-5-trifluoromethylsulfanyl-benzothiazole-2-carboxylic acid amide.

### CHEMICAL SYNTHESIS

The compounds of the present invention can be prepared by any method known in the art. In particular, benzthiazole-3-oxides **1** can be obtained from nitrobenzenes **2,** in which Z is a suitable leaving group, such as halogen or tosyl, by reaction with thioglycolic acid esters **3.** Such aromatic nucleophilic substitution can be achieved readily when R² and/or R⁴ are substituents that activate such reaction. Alternatively, palladium-catalysed substitution can be employed (Migita et al., Bull. Chem. Soc. Jpn., 1980, 53, 1385). Under the influence of excess tertiary amine base, the initially formed thioanisole adducts spontaneously cyclise to the benzthiazole-3-oxide carboxylic acid esters **4** or cyclisation can be induced by heating (Wagner et al., Chem. Ber., 1973, 106, 640). The ester function can be transformed in various ways to obtain the desired substituents R⁵. Thus, carboxamides (**1,** R⁵ = CONR⁶R⁷) can be prepared by treatment of esters **5** with the corresponding amines, which in turn can be transformed into nitriles (**1,** R⁵ = CN), or alcohols (**1,** R⁵ = OH) (Wagner et al., Chem. Ber., 1976, 109, 611). Carboxylic acid hydroxamides (1, R⁵ = CONHOH) and hydrazides (1, R⁵ = CONHNH₂) can be obtained similarly.

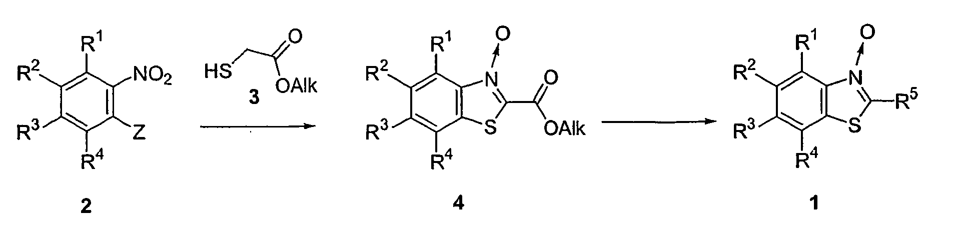

### ASSAYS

Another aspect of the invention relates to the use of a compound of formula I, or a pharmaceutically acceptable salt thereof, as defined hereinabove in an assay for identifying further candidate compounds that influence the activity of one or more polo-like kinases.

Preferably, the assay is capable of identifying candidate compounds that are capable of inhibiting one or more polo-like kinases.

More preferably, the assay is a competitive binding assay.

As used herein, the term "candidate compound" includes, but is not limited to, a compound which may be obtainable from or produced by any suitable source, whether natural or not.

The candidate compound may be designed or obtained from a library of compounds, which may comprise peptides, as well as other compounds, such as small organic molecules and particularly new lead compounds. By way of example, the candidate compound may be a natural substance, a biological macromolecule, or an extract made from biological materials - such as bacteria, fungi, or animal (particularly mammalian) cells or tissues, an organic or an inorganic molecule, a synthetic candidate compound, a semi-synthetic candidate compound, a structural or functional mimetic, a peptide, a peptidomimetic, a derivatised candidate compound, a peptide cleaved from a whole protein, or a peptide synthesised synthetically, for example, either using a peptide synthesiser or by recombinant techniques or combinations thereof, a recombinant candidate compound, a natural or a non-natural candidate compound, a fusion protein or equivalent thereof and mutants, derivatives or combinations thereof. The candidate compound may even be a compound that is a modulator of PLK, such as a known inhibitor of PLK, that has been modified in some way eg. by recombinant DNA techniques or chemical synthesis techniques.

Typically, the candidate compound will be prepared by recombinant DNA techniques and/or chemical synthesis techniques.

Once a candidate compound capable of interacting PLK has been identified, further steps may be carried out to select and/or to modify the candidate compounds and/or to modify existing compounds, such that they are able to modulate PLK.

Preferably, the candidate compound is generated by conventional SAR modification of a compound of the invention.

As used herein, the term "conventional SAR modification" refers to standard methods known in the art for varying a given compound by way of chemical derivatisation.

Thus, in one aspect, the identified compound may act as a model (for example, a template) for the development of other compounds. The compounds employed in such a test may be free in solution, affixed to a solid support, borne on a cell surface, or located intracellularly. The abolition of activity or the formation of binding complexes between the compound and the agent being tested may be measured.

The assay of the present invention may be a screen, whereby a number of agents are tested. In one aspect, the assay method of the present invention is a high through-put screen.

This invention also contemplates the use of competitive drug screening assays in which neutralising antibodies capable of binding a compound specifically compete with a candidate compound for binding to a compound.

Another technique for screening provides for high throughput screening (HTS) of agents having suitable binding affinity to the substances and is based upon the method described in detail in WO 84/03564.

It is expected that the assay methods of the present invention will be suitable for both small and large-scale screening of test compounds as well as in quantitative assays.

The invention can find utility in a process comprising the steps of:
(a) performing an assay method described hereinabove;
(b) identifying one or more candidate compounds capable of binding to a polo-like kinase; and
(c) preparing a quantity of said one or more candidate compounds.

Another aspect of the invention provides a process comprising the steps of:
(a) performing an assay method described hereinabove;
(b) identifying one or more candidate compounds capable of binding to a polo-like kinase; and
(c) preparing a pharmaceutical composition comprising said one or more candidate compounds.

Another aspect of the invention provides a process comprising the steps of:
(a) performing an assay method described hereinabove;
(b) identifying one or more candidate compounds capable of binding to a polo-like kinase;
(c) modifying said one or more candidate compounds capable of binding to a polo-like kinase;
(d) performing the assay method described hereinabove;
(e) optionally preparing a pharmaceutical composition comprising said one or more candidate compounds.

The present invention is further described by way of example.

### EXAMPLES

### Example 1

The example compounds of the present invention are listed in Table 1. Known compounds of structure 1 were prepared as described (Wagner et al., Chem. Ber., 1973, 106, 640; *ibid.* 1974, 107, 305; *ibid.* 1976, 109, 611). Compounds with novel chemical composition (**1b, 1f, 1g,** and **1j**) were prepared similarly.

For compounds **1a - 1e** and **1h - 1j,** triethylamine (1.1 eq) was added to a suspension of the appropriate 2-chloro-1,3-dinitro-benzene precursor (1 eq) and mercapto-acetic acid ethyl ester (1 eq) in ethanol (3 mL) at 10 - 20 °C. Onset of reaction was indicated by an exotherm. The mixture was stirred with ice-bath cooling for an additional 3 h. The precipitated 7-nitro-3-oxy-benzothiazole-2-carboxylic acid ethyl ester products were collected by filtration and they were recrystallised from methanol.

For compounds **1f** and **1g,** 4-fluoro-2,6-dinitrophenol (1 eq) was dissolved in toluene (5 mL) and tosyl chloride (1.1 eq) was added, followed by pyridine (1.1 eq). The mixture was stirred at room temperature for 12 h. After filtration, the filtrate was evaporated *in vacuo* and the residue of toluene-4-sulfonic acid 4-fluoro-2,6-dinitro-phenyl ester was used without further purification in the reaction with mercapto-acetic acid ethyl ester reaction as described above.

The appropriate 7-nitro-3-oxy-benzothiazole-2-carboxylic acid ethyl ester products were further reacted in methanol with excess ammonium hydroxide (for **1a, 1d, 1f,** and **1j**), pyrrolidine (for **1b**), hydrazine (for **1c**), or hydroxylamine (**1**e and **1g**) for 4 h at room temperature. Products were filtered, washed with water and methanol, dried, and recrystallised from methanol.

Compound **1h** was obtained from **1a** by successive treatment with excess phosphorous oxychloride of a solution in pyridine during several hours. After dilution with water, the products were filtered, washed with water, dried, and recrystallised from methanol.

Compound **1i** was obtained from **1h** by treatment with excess aqueous sodium hydroxide solution of a solution in water-methanol. After stirring at room temperature for 3 h, the clear solution was acidified and the precipitated product was filtered, washed with water and methanol, and was recrystallised from methanol.

Analytical data for the compounds in Table 1 are given below. Melting ponts (mp) are not corrected. HPLC analysis was performed using Vydac 218TP54 (4.5 × 250 mm) C₁₈ reversed-phase silica columns. Elution was at 1 mL/min using a linear gradient from 10 to 70 % acetonitrile in 0.1 % aq trifluoroacetic acid over 20 min. Purities were obtained from peak integration of chromatograms (monitoring at λ = 254 nm). ¹H-NMR spectra were obtained at 500 MHz field strength; chemical shifts are expressed in p.p.m. relative to tetramethylsilane internal standard.

### 7-Nitro-3-oxy-5-trifluoromethyl-benzothiazole-2-carboxylic acid amide (1a)

¹H-NMR (DMSO-d₆) δ: 8.76 (2H, s, Ar-H and NH), 8.96 (1H, s, Ar-H), 9.36 (1H, s, NH); mp 222-223 °C; HPLC: t*_{R}* 13.75 min (100 %).

### (7-Nitro-3-oxy-5-trifluoromethyl-benzothiazol-2-yl)-pyrrolidin-1-yl-methanone (1b)

¹H-NMR (CDCl₃) δ: 2.04 (4H, t, *J* = 3.5 Hz, CH₂), 3.76 (4H, dt, *J* = 18.0, 3.5 Hz, CH₂), 8.21 (1H, s, Ar-H), 8.84 (1H, s, Ar-H); mp 176-178 °C; HPLC: t*_{R}* 14.17 min (100 %).

### 7-Nitro-3-oxy-5-trifluoromethyl-benzothiazole-2-carboxylic acid hydrazide (1c)

¹H-NMR (DMSO-d₆) δ: 5.20 (2H, s, NH₂), 8.88 (1H, s, Ar-H), 8.95 (1H, s, Ar-H), 11.20 (1H, s, NH); mp 229-230 °C; HPLC: t*_{R}* 11.96 min (100 %).

### 5-Cyano-7-nitro-3-oxy-benzothiazole-2-carboxylic acid amide (1d)

¹H-NMR (DMSO-d₆) δ: 8.86 (1H, s, NH), 9.16 (1H, s, Ar-H), 9.22 (1H, s, Ar-H), 9.33 (1H, s, NH); mp 255-258 °C; HPLC: t*_{R}* 9.30 min (78 %).

### 7-Nitro-3-oxy-5-trifluoromethyl-benzothiazole-2-carboxylic acid hydroxyamide (1e)

¹H-NMR (DMSO-d₆) δ: 8.86, 8.96 (2H, s, Ar-H), 10.17, 12.13 (2H, s, OH and NH); mp 230-231 °C; MS: *m*/*z* (%) 346 (MNa⁺, 100) and 324 (MH⁺, 63); HPLC: t_{R} 12.33 min (94 %).

### 5-Fluoro-7-nitro-3-oxy-benzothiazole-2-carboxylic acid hydroxyamide (1g)

¹H-NMR (DMSO-d₆) δ: 8.59, 8.79 (2H, s, Ar-H), 10.16, 12.17 (2H, s, OH and NH); mp 224-225 °C; HPLC: t*_{R}* 8.62 min (100 %).

### 7-Nitro-3-oxy-5-trifluoromethyl-benzothiazole-2-carbonitrile (1h)

¹H-NMR (DMSO-d₆) δ: 8.91 (1H, s, Ar-H), 9.02 (1H, s, Ar-H); mp 188-189 °C; HPLC: t*_{R}* 15.60 min (100 %).

### 7-Nitro-3-oxy-5-trifluoromethyl-benzothiazol-2-ol (1i)

¹H-NMR (DMSO-d₆) δ: 7.88 (1H, s, Ar-H), 8.35 (1H, s, Ar-H); mp 181-183 °C; HPLC: t*_{R}* 16.37 min (85 %).

### 7-Nitro-3-oxy-5-trifluoromethylsulfanyl-benzothiazole-2-carboxylic acid amide (1j)

¹H-NMR (DMSO-d₆) δ: 8.82, 8.85, 8.90 (3H, s, Ar-H and NH), 9.35 (1H, s, NH); mp 255-256 °C; HPLC t_{R} 15.79 min (100 %).

**Table 1. Structures of example compounds**

| **No.** | **Name** | **Formula** | 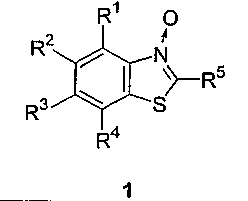 | | | | |
|---|---|---|---|---|---|---|---|
| | | | **R¹** | **R²** | **R³** | **R⁴** | **R⁵** |
| **1a** | 7-Nitro-3-oxy-5-trifluoromethyl-benzothiazole-2-carboxylic acid amide | 1 | H | CF₃ | H | NO₂ | CONH₂ |
| **1b** | (7-Nitro-3-oxy-5-trifluoromethyl-benzothiazol-2-yl)-pyrrolidin-1-yl-methanone | 1 | H | CF₃ | H | NO₂ | CO-pyrrolidine |
| **1c** | 7-Nitro-3-oxy-5-trifluoromethyl-benzothiazole-2-carboxylic acid hydrazide | 1 | H | CF₃ | H | NO₂ | CONHNH₂ |
| **1d** | 5-Cyano-7-nitro-3-oxy-benzothiazole-2-carboxylic acid amide | 1 | H | CN | H | NO₂ | CONH₂ |
| **1e** | 7-Nitro-3-oxy-5-trifluoromethyl-benzothiazole-2-carboxylic acid hydroxyamide | 1 | H | CF₃ | H | NO₂ | CONHOH |
| **1f** | 5-Fluoro-7-nitro-3-oxy-benzothiazole-2-carboxylic acid amide | 1 | H | F | H | NO₂ | CONH₂ |
| **1g** | 5-Fluoro-7-nitro-3-oxy-benzothiazole-2-carboxylic acid hydroxyamide | 1 | H | F | H | NO₂ | CONHOH |
| **1h** | 7-Nitro-3-oxy-5-trifluoromethyl-benzothiazole-2-carbonitrile | 1 | H | CF₃ | H | NO₂ | CN |
| **1i** | 7-Nitro-3-oxy-5-trifluoromethyl-benzothiazol-2-ol | 1 | H | CF₃ | H | NO₂ | OH |
| **1j** | 7-Nitro-3-oxy-5-trifluoromethylsulfanyl-benzothiazole-2-carboxylic acid amide | 1 | H | SCF₃ | H | NO₂ | CONH₂ |

### Example 2

A full-length human CDC25C clone was isolated by PCR from HeLa mRNA and was inserted on a BamHI-HindIII fragment into pRsetA. The amino terminal fragment of CDC25C (encoding residues 1-300) was excised from this vector and inserted into pET28a (between the NcoI and BamHI sites). Expression was under the control of the T7 promoter, and the encoded protein contained a His₆ tag at the carboxyl terminus. The vector was transformed into *E*. *coli* strain BRL(DE3) pLysS for expression experiments. CDC25C was expressed in BL21(DE3) RIL bacteria cells, grown in LB media at 37 °C until OD₆₀₀ₙₘ of 0.6 was reached. The expression was induced with 1 mM IPTG and the bacterial culture was grown further for 3 h. The bacteria were harvested by centrifugation and the cell pellet was re-suspended in 50 mM Tris pH 7.5 and 10 % sucrose, flash frozen, and stored at -70 °C until used. CDC25C protein was purified from *E*. *coli* inclusion bodies. The inclusion bodies were isolated in a buffer (50 mM Tris pH 8.0, 2 mM EDTA, 100 mM NaCl, 0.5 % triton X-100). After denaturation in the presence of 6 M urea, the protein was refolded via slow dialysis of the urea. The protein was stored in 25 mM Tris pH 8.0, 100 mM NaCl, 1 mM DTT, 1 mM EDTA and 10 % glycerol at -70 °C until used.

A full-length human PLK1 (XM_047240) (amino acids 1-603) clone was amplified from a foetal lung cDNA library using primers incorporating restriction enzyme sites. The 5' primer (gccgctagcgacgatgacgataagatgagtgctgcagtgactgcagggaagc) had an Nhe1 site prior to the ATG start codon. The 3' primer (ggaattcttaggaggccttgagacgg) incorporated a stop codon prior to the EcoR1 site. The PCR product was cloned into the Nhel/EcoRl sites of a baculovirus expression vector, pSSP1. Cloning into this vector resulted in a His₆ tag fusion at the amino terminus of the PLK1 construct. Sf9 cells of a passage number less than 20 were split back to give a 300 mL culture volume, at a cell density of 1.5 × 10⁶ cells/mL. Cells were only used for expression in logarithmic growth phase. PLK1 baculovirus (from P2 amplification) was added to give a multiplicity of infection of 3, this is equivalent to 3 virus particles for each insect cell. The flasks were incubated at 27 °C, with shaking at 100 r.p.m. for 48 h. On harvest, cell density and viability was determined, the cultures spun down at 2500 r.p.m. for 5 min and washed with ice-cold phosphate-buffered saline. The wash was re-spun at the same speed and the pellet was snap frozen. PLK1 protein was purified on a metal affinity column. The insect cell pellet was lysed in a buffer (10 mM Tris-HCl pH 8.0, 150 mM NaCl, 5 mM β-mercaptoethanol, 1 mM PMSF, 1 mM benzamidine, 20 mM imidazole and protease inhibitor cocktail (Sigma) and the pre-cleared supernatant was loaded onto NiNTA-agarose (Qiagen). The affinity column was washed with the lysis buffer and the bound protein was eluted with 250 mM imidazole in the same buffer. After overnight dialysis against 25 mM Tris HCl, pH 7.5, 100 mM NaCl, 1 mM DTT, 1 mM PMSF, 1 mM benzamidine, protease inhibitors cocktail (Sigma) and 10 % glycerol, the purified protein was stored at -70°C until used.

### Example 3

PLK1 protein kinase assays were carried out using a 96-well plate format by incubating CDC25C (2 µg/well) with PLK1 (1 µg/well) in 20 mM Tris/HCl buffer pH 7.5, supplemented with 25 mM β-glycerophosphate, 5 mM EGTA, 1 mM DTT and 1 mM NaVO₃. Serial dilutions of test compound in assay buffer were added. Reaction was initiated by the addition of 100 µM ATP and 0.5 µCi of [γ-³²P]-ATP. The reaction mixture was incubated at 30 °C for 1 h, then stopped with 75 mM aq orthophosphoric acid, transferred onto a 96-well P81 filter plate (Whatman), dried, and the extent of CDC25C phosphorylation was assessed by scintillation counting using a Packard TopCount plate reader. The raw assay data was analysed by non-linear regression analysis parameters and IC₅₀ values were determined using the equation: y = A + ((B-A)/(1+((C/x)^D))), where y is % inhibition, A is minimum inhibition, B is maximum inhibition, C is EC₅₀, and D is the slope factor.

Cellular proliferation assays using human tumour cell lines (obtained from the American Type Culture Collection, 10801 University Boulevard, Manessas, VA 20110-2209, USA) were carried out. Standard 72-h MTT (thiazolyl blue; 3-[4,5-dimethylthiazol-2-yl]-2,5-diphenyltetrazolium bromide) assays were performed (Loveland et al, Biochem. Int., 1992, 27, 501; Haselsberger et al, Anti Cancer Drugs, 1996, 7, 331). In short: cells were seeded into 96-well plates according to doubling time and incubated overnight at 37 °C. Test compounds were made up in DMSO and a 1/3 dilution series prepared in 100 µL cell media, added to cells (in triplicates) and incubated for 72 h at 37 °C. MTT was made up as a stock of 5 mg/mL in cell media and filter-sterilised. Media was removed from cells followed by a wash with 200 µL PBS. MTT solution was then added at 20 µL per well and incubated in the dark at 37 °C for 4 h. MTT solution was removed and cells again washed with 200 µL PBS. MTT dye was solubilised with 200 µL per well of DMSO with agitation. Absorbance was read at 540 nm and data analysed using curve-fitting software (GraphPad Prism version 3.00 for Windows, GraphPad Software, San Diego California USA) to determine IC₅₀ values (concentration of test compound which inhibits cell growth by 50%).

Inhibition data for the example compounds of this invention are summarised in Table 2.

**Table 2. Biological activity.**

| | **IC₅₀ (µM)** | | | | |
|---|---|---|---|---|---|
| **Compound No.*** | **PLK1 kinase** | **Anti-proliferative activity on tumour cell lines (MTT assay)** | | | |
| | **inhibition** | *A549* | *HELA* | *MCF-7* | *U2OS* |
| **1a** | 2.4 ± 1.4 | 24 ± 3 | 16 ± 8 | 12.6 ± 6.3 | 47 ± 7 |
| **1b** | 56 ± 19 | 5.2 | 12 | n/d*** | 16 |
| **1c** | >100 | 21 ± 3 | 16 ± 10 | 19 ± 16 | 35 ± 3 |
| **1d** | 10 ± 7 | 4.2 ± 1.5 | 6.6 ± 0.8 | 2 ± 2 | 6.7 ± 1.2 |
| **1e** | 50-100 | 13.3 ± 0.6 | 21 ± 4 | 9 ± 1 | 29.4 ± 1.2 |
| **1f** | 50-100** | 3.9 ± 0.2 | 10.2 ± 2.4 | 1.6 ± 0.2 | 11.5 ± 2.0 |
| **1g** | 67 ± 7 | 7.7 ± 2.9 | 14 ± 5 | 6.1 ± 1.1 | 7.2 ± 3.6 |
| **1h** | 21 | 10.0 ± 0.08 | 10.2 ± 3.3 | 2.1 ± 1.5 | 9.5 ± 1.7 |
| **1i** | 39 | 48 ± 5 | 56 ± 2 | 35 ± 2 | 88 ± 10 |
| **1j** | 0.06 ± 0.03 | 4.0 ± 1.8 | 6.0 ± 0.8 | 2.5 ± 1.0 | 8.2 ± 0.3 |

| | | | | | |
|---|---|---|---|---|---|
| * Refer Table 1. ** This result was obtained performing the kinase assay at 10 µM ATP in contrast to others performed at 100 µM ATP. *** n/d: Not determined. | | | | | |

Various modifications and variations of the described aspects of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the relevant fields are intended to be within the scope of the following claims.

## Claims

1. Use of a compound of formula I, or a pharmaceutically acceptable salt thereof, 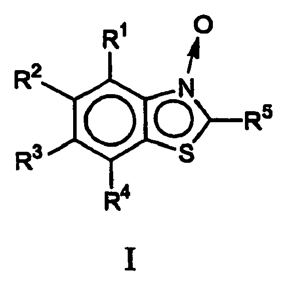 wherein
R¹, R², R³, and R⁴ are each independently H, NO₂, CF₃, SCF₃, CN, halo, OH, OR⁶, NH₂, NHR⁶, NR⁶R⁷, N⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶, SO₂NH₂, SO₂NHR⁶, SO₂NR⁶R⁷, alkyl, cycloalkyl, cycloheteroalkyl, aryl, or heteroaryl, with the proviso that at least one of R¹, R², R³, and R⁴ is other than H; or R¹ and R², R² and R³, or R³ and R⁴, may together form part of a fused or unfused saturated or unsaturated ring system, optionally containing up to two heteroatoms selected from N, O, and S;
R⁵ is OH, OR⁹, CN, CONH₂, CONHNH₂, CONHOH, CONHR⁹, CONR⁹R¹⁰, NH₂, NHR⁹, or NR⁹R¹⁰;
each R⁶, R⁷ and R⁸ is independently hydrocarbyl, or two of R⁶, R⁷ and R⁸ together form part of a saturated or unsaturated ring system, optionally containing up to two heteroatoms selected from N, O, and S;
each R⁹ and R¹⁰ is independently hydrocarbyl, or R⁹ and R¹⁰ together form part of a saturated or unsaturated ring system, optionally containing up to two heteroatoms selected from N, O, and S;
in the preparation of a medicament for treating a proliferative disorder.

2. Use according to claim 1 wherein R¹, R², R³, and R⁴ are each independently H, NO₂, CF₃, SCF₃, CN, halo, OH, OR⁶, NH₂, NHR⁶, NR⁶R⁷, N⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶, SO₂NH₂, SO₂NHR⁶, SO₂NR⁶R⁷, alkyl, cycloalkyl, cycloheteroalkyl, aryl, or heteroaryl.

3. Use according to claim 1 or claim 2 wherein each R⁶, R⁷ and R⁸ is independently hydrocarbyl, or two of R⁶, R⁷ and R⁸ together form part of a saturated ring system, optionally containing up to two heteroatoms selected from N, O, and S.

4. Use according to any preceding claim wherein each R⁹ and R¹⁰ is independently hydrocarbyl, or R⁹ and R¹⁰ together form part of a saturated ring system, optionally containing up to two heteroatoms selected from N, O, and S.

5. Use according to any preceding claim wherein the hydrocarbyl group is an alkyl or aryl group.

6. Use according to any preceding claim wherein R⁵ is OH, CN, CONH₂, CONHNH₂, CONHOH or CONR⁹R¹⁰.

7. Use according to claim 6 wherein R⁹ and R¹⁰ together form part of a saturated ring system.

8. Use according to any preceding claim wherein R⁵ is selected from CONH₂, CO-pyrrolidine, CONHNH₂, CONHOH, CN and OH.

9. Use according to any preceding claim wherein R¹, R², R³, and R⁴ are each independently H, NO₂, CF₃, SCF₃ or halo.

10. Use according to any preceding claim wherein R² is selected from CF₃, F, SCF₃ and H.

11. Use according to any preceding claim wherein R³ and R¹ are both H.

12. Use according to any preceding claim wherein R⁴ is NO₂.

13. Use according to any preceding claim wherein said compound of formula I is selected from the following:
7-Nitro-3-oxy-5-trifluoromethyl-benzothiazole-2-carboxylic acid amide;
(7-Nitro-3-oxy-5-trifluoromethyl-benzothiazol-2-yl)-pyrrolidin-1-yl-methanone;
7-Nitro-3-oxy-5-trifluoromethyl-benzothiazole-2-carboxylic acid hydrazide;
5-Cyano-7-nitro-3-oxy-benzothiazole-2-carboxylic acid amide;
7-Nitro-3-oxy-5-trifluoromethyl-benzothiazole-2-carboxylic acid hydroxyamide;
5-Fluoro-7-nitro-3-oxy-benzothiazole-2-carboxylic acid amide;
5-Fluoro-7-nitro-3-oxy-benzothiazole-2-carboxylic acid hydroxyamide;
7-Nitro-3-oxy-5-trifluoromethyl-benzothiazole-2-carbonitrile;
7-Nitro-3-oxy-5-trifluoromethyl-benzothiazol-2-ol; and
7-Nitro-3-oxy-5-trifluoromethylsulfanyl-benzothiazolo-2-carboxylic acid amide.

14. Use according to claim 13 wherein the compound of formula I is selected from the following:
7-Nitro-3-oxy-5-trifluoromethyl-benzothiazole-2-carboxylic acid amide;
5-Cyano-7-nitro-3-oxy-benzothiazole-2-carboxylic acid amide;
7 -Nitro-3-oxy-5-trifluoromethyl-benzothiazole--2-carbonitrile; and
7-Nitro-3-oxy-5-trifluoromethylsulfanyl-benzothiazolo-2-carboxylic acid amide.

15. Use according to claim 13 wherein the compound of formula I is selected from the following:
(7-Nitro-3-oxy 5-trifluoromethyl-benzothiazol-2-yl)-pyrrolidin-1-yl-methanone;
5-Cyano-7-nitro-3-oxy benzothiazole-2-carboxylic acid amide;
5-Fluoro-7-nitro-3-oxy-benzothiazole-2-carboxylic acid amide;
5-Fluoro-7-nitro-3-oxy-benzothiazole-2-carboxylic acid hydroxyamide;
7-Nitro-3-oxy-5-trifluoromethyl-benzothiazole-2-carbonitrile; and
7-Nitro-3-oxy-5-trifluoromethylsulfanyl-benzothiazole-2-carboxylic acid amide.

16. Use according to any preceding claim wherein the proliferative disorder is cancer.

17. Use according to any one of claims 1 to 15 wherein the proliferative disorder is glomerulonephritis.

18. Use according to any one of claims 1 to 15 wherein the proliferative disorder is rheumatoid arthritis.

19. Use according to any one of claims 1 to 15 wherein the proliferative disorder is psoriasis.

20. Use according to any one of claims 1 to 15 wherein the proliferative disorder is a chronic obstructive pulmonary disorder.

21. Use according to any preceding claim wherein said compound of formula I is administered in an amount sufficient to inhibit at least one PLK enzyme.

22. Use according to claim 21 wherein the PLK enzyme is PLK1.

23. Use according to any preceding claim wherein said compound of formula I is administered in combination with a pharmaceutically acceptable excipient, diluent or carrier.

24. Use according to any preceding claim wherein said compound of formula I is administered in combination with a further anti-proliferative agent.

25. A compound selected from the following:
(7-Nitro-3-oxy-5-trifluoromethyl-benzothiazol-2-yl)-pyrrolidin-1-yl-methanone;
5-Fluoro-7-nitro-3-oxy-benzothiazole-2-carboxylic acid amide;
5-Fluoro-7-nitro-3-oxy-benzothiazole-2-carboxylic acid hydroxyamide; and
7-Nitro-3-oxy-5-trifluoromethylsulfanyl-benzothiazole-2-carboxylic acid amide.

26. A pharmaceutical composition comprising a compound according to claim 25 admixed with a pharmaceutically acceptable diluent, excipient or carrier.

27. Use of a compound of formula I as defined in any one of claims 1 to 15 in an assay for identifying further candidate compounds capable of inhibiting PLK.

28. Use according to claim 27 wherein said assay is a competitive binding assay.

## Patentansprüche

1. Verwendung einer Verbindung der Formel I oder eines pharmazeutisch verträglichen Salzes davon 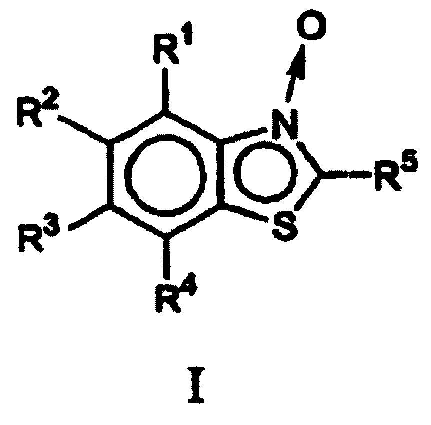 wobei
R¹, R², R³ und R⁴ jeweils unabhängig voneinander H, NO₂, CF₃, SCF₃, CN, Halogen, OH, OR⁶, NH₂, NHR⁶ NR⁶R⁷, N⁺R⁶R⁷R⁸, COOH, COOR⁶, CONH₂, CONHR⁶, CONR⁶R⁷, COH, COR⁶ SR⁶, SOR⁶, SO₂R⁶, SO₂OH, SO₂OR⁶, SO₂NH₂, SO₂NHR⁶, SO₂NR⁶R⁷ , Alkyl, Cycloalkyl, Cycloheteroalkyl, Aryl oder Heteroaryl sind, mit der Maßgabe, daß wenigstens einer von R¹, R², R³ und R⁴ etwas anderes als H ist, oder R¹ und R², R² und R³ oder R³ und R⁴ zusammen Teil eines kondensierten oder nicht kondensierten gesättigten oder ungesättigten Ringsystems sein können, welches optional bis zu zwei Heteroatome, ausgewählt unter N, O und S, enthält,
R⁵ OH, OR⁹, CN, CONH₂, CONHNH₂, CONHOH, CONHR⁹, CONR⁹R¹⁰, NH₂, NHR⁹ oder NR⁹R¹⁰ ist,
jeder R⁶, R⁷ und R⁸ unabhängig voneinander Hydrocarbyl ist oder zwei von R⁶, R⁷ und R⁸ zusammen Teil eines gesättigten oder ungesättigten Ringsystems bilden, welches optional bis zu zwei Heteroatome, ausgewählt unter N, O und S, enthält,
jeder R⁹ und R¹⁰ unabhängig voneinander Hydrocarbyl ist oder R⁹ und R¹⁰ zusammen Teil eines gesättigten oder ungesättigten Ringsystems bilden, welches optional bis zu zwei Heteroatome, ausgewählt unter N, O und S, enthält,
bei der Herstellung eines Medikaments zur Behandlung einer proliferativen Störung.

2. Verwendung nach Anspruch 1, wobei R¹, R², R³ und R⁴ jeweils unabhängig voneinander H, NO₂, CF₃, SCF₃, CN, Halogen, OH, OR⁶, NH₂, NHR⁶, NR⁶R⁷, N⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶, SO₂NH₂, SO₂NHR⁶, SO₂NR⁶R⁷, Alkyl, Cycloalkyl, Cycloheteroalkyl, Aryl oder Heteroaryl sind.

3. Verwendung nach Anspruch 1 oder Anspruch 2, wobei jeder R⁶, R⁷ und R⁸ unabhängig voneinander Hydrocarbyl ist oder zwei von R⁶, R⁷ und R⁸ zusammen Teil eines gesättigten Ringsystems bilden, welches optional bis zu zwei Heteroatome, ausgewählt unter N, O und S, enthält.

4. Verwendung nach einem der vorangegangenen Ansprüche, wobei jeder R⁹ und R¹⁰ unabhängig voneinander Hydrocarbyl ist oder R⁹ und R¹⁰ zusammen Teil eines gesättigten Ringsystems bilden, welches optional bis zu zwei Heteroatome, ausgewählt unter N, O und S, enthält.

5. Verwendung nach einem der vorangegangenen Ansprüche, wobei die Hydrocarbylgruppe eine Alkyl- oder Arylgruppe ist.

6. Verwendung nach einem der vorangegangenen Ansprüche, wobei R⁵ OH, CN, CONH₂, CONHNH₂, CONHOH oder CONR⁹R¹⁰ ist.

7. Verwendung nach Anspruch 6, wobei R⁹ und R¹⁰ zusammen Teil eines gesättigten Ringsystems bilden.

8. Verwendung nach einem der vorangegangenen Ansprüche, wobei R⁵ unter CONH₂, CO-Pyrrolidin, CONHNH₂, CONHOH, CN und OH ausgewählt ist.

9. Verwendung nach einem der vorangegangenen Ansprüche, wobei R¹, R², R³ und R⁴ jeweils unabhängig voneinander H, NO₂, CF₃, SCF₃ oder Halogen sind.

10. Verwendung nach einem der vorangegangenen Ansprüche, wobei R² unter CF₃, F, SCF₃ und H ausgewählt ist.

11. Verwendung nach einem der vorangegangenen Ansprüche, wobei R³ und R¹ beide H sind.

12. Verwendung nach einem der vorangegangenen Ansprüche, wobei R⁴ NO₂ ist.

13. Verwendung nach einem der vorangegangenen Ansprüche, wobei die Verbindung der Formel 1 unter den folgenden ausgewählt ist:
7-Nitro-3-oxy-5-trifluormethyl-benzothiazol-2-carbonsäureamid,
(7-Nitro-3-oxy-5-trifluormethyl-benzothiazol-2-yl)-pyrrolidin-1-yl-methanon,
7-Nitro-3-oxy-5-trifluormethyl-benzothiazol-2-carbonsäurehydrazid,
5-Cyano-7-nitro-3-oxy-benzothiazol-2-carbonsäureamid,
7-Nitro-3-oxy-5-trifluromethyl-benzothiazol-2-carbonsäurehydroxyamid,
5-Fluor-7-nitro-3-oxy-benzothiazol-2-carbonsäureamid,
5-Fluor-7-nitro-3-oxy-benzothiazol-2-carbonsäurehydroxyamid,
7-Nitro-3-oxy-5-trifluormethyl-benzothiazol-2-carbonitril,
7-Nitro-3-oxy-5-trifluormethyl-benzothiazol-2-ol und
7-Nitro-3-oxy-5-trifluormethylsulfanyl-benzothiazol-2-carbonsäureamid.

14. Verwendung nach Anspruch 13, wobei die Verbindung der Formel I unter den folgenden ausgewählt ist:
7-Nitro-3-oxy-5-trifluormethyl-benzothiazol-2-carbonsäureamid,
5-Cyano-7-nitro-3-oxy-benzothiazol-2-carbonsäureamid,
7-Nitro-3-oxy-5-trifluormethyl-benzothiazol-2-carbonitril und
7-Nitro-3-oxy-5-trifluormethylsulfanyl-benzothiazol-2-carbonsäureamid.

15. Verwendung nach Anspruch 13, wobei die Verbindung der Formel I unter den folgenden ausgewählt ist:
(7-Nitro-3-oxy-5-trifluormethyl-benzothiazol-2-yl)-pyrrolidin-1-yl-methanon,
5-Cyano-7-nitro-3-oxy-benzothiazol-2-carbonsäureamid,
5-Fluor-7-nitro-3-oxy-benzothiazol-2-carbonsäureamid,
5-Fluor-7-nitro-3-oxy-benzothiazol-2-carbonsäurehydroxyamid,
7-Nitro-3-oxy-5-trifluormethyl-benzothiazol-2-carbonitril und
7-Nitro-3-oxy-5-trifluormethylsulfanyl-benzothiazol-2-carbonsäureamid.

16. Verwendung nach einem der vorangegangenen Ansprüche, wobei die proliferative Störung Krebs ist.

17. Verwendung nach einem der Ansprüche 1 bis 15, wobei die proliferative Störung Glomerulonephritis ist.

18. Verwendung nach einem der Ansprüche 1 bis 15, wobei die proliferative Störung rheumatoide Arthritis ist.

19. Verwendung nach einem der Ansprüche 1 bis 15, wobei die proliferative Störung Psoriasis ist.

20. Verwendung nach einem der Ansprüche 1 bis 15, wobei die proliferative Störung eine chronisch obstruktive Lungenerkrankung ist.

21. Verwendung nach einem der vorangegangenen Ansprüche, wobei die Verbindung der Formel I in einer Menge verabreicht wird, die ausreichend ist, um wenigstens ein PLK-Enzym zu hemmen.

22. Verwendung nach Anspruch 21, wobei das PLK-Enzym PLK1 ist.

23. Verwendung nach einem der vorangegangenen Ansprüche, wobei die Verbindung der Formel I in Kombination mit einem pharmazeutisch verträglichen Hilfsstoff, Verdünnungsmittel oder Träger verabreicht wird.

24. Verwendung nach einem der vorangegangenen Ansprüche, wobei die Verbindung der Formel I in Kombination mit einem weiteren antiproliferativen Mittel verabreicht wird.

25. Verbindung, ausgewählt unter den folgenden:
(7-Nitro-3-oxy-5-trifluormethyl-benzothiazol-3-yl)-pyrrolidin-1-yl-methanon,
5-Fluor-7-nitro-3-oxy-benzothiazol-2-carbonsäureamid,
5-Fluor-7-nitro-3-oxy-benzothiazol-2-carbonsäurehydroxyamid und
7-Nitro-3-oxy-5-trifluormethylsulfanyl-benzothiazol-2-carbonsäureamid.

26. Pharmazeutische Zusammensetzung, welche eine Verbindung nach Anspruch 25 im Gemisch mit einem pharmazeutisch verträglichen Verdünnungsmittel, Hilfsstoff oder Träger umfaßt.

27. Verwendung einer Verbindung der Formel 1, wie sie in einem der Ansprüche 1 bis 15 definiert ist, in einem Test zum Identifizieren weiterer Kandidatenverbindungen, die PLK hemmen können.

28. Verwendung nach Anspruch 27, wobei der Test ein kompetitiver Bindungstest ist.

## Revendications

1. Utilisation d'un composé de formule I, ou d'un de ses sels pharmaceutiquement acceptables, 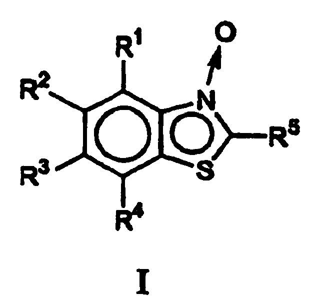 formule dans laquelle
R¹, R², R³ et R⁴ représentent chacun indépendamment H, un groupe NO₂, CF₃, SCF₃, CN, halogéno, OH, OR⁶, NH₂, NHR⁶, NR⁶R⁷, R⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶ , SO₂NH₂, SO₂NHR⁶ , SO₂NR⁶R⁷, alkyle, cycloalkyle, cyclohétéroalkyle, aryle ou hétéroaryle, sous réserve qu'au moins un des groupes R¹, R², R³ et R⁴ soit autre que H ; ou bien R¹ et R², R² et R³ ou R³ et R⁴ peuvent former conjointement une partie d'un système de noyau saturé ou insaturé, condensé ou non condensé, contenant facultativement jusqu'à deux hétéroatomes choisis entre N, O et S ;
R⁵ représente un groupe OH, OR⁹, CN, CONH₂, CONHNH₂, CONHOH, CONHR⁹, CONR⁹R¹⁰ NH₂, NHR⁹ ou NR⁹R¹⁰ ;
chacun des groupes R⁶ R⁷ et R⁸ représente indépendamment un groupe hydrocarbyle, ou bien deux des groupes R⁶, R⁷ et R⁸ forment conjointement une partie d'un système de noyau saturé ou insaturé, contenant facultativement jusqu'à deux hétéroatomes choisis entre N, O et S ;
chacun des groupes R⁹ et R¹⁰ représente indépendamment un groupe hydrocarbyle, ou bien R⁹ et R¹⁰ forment conjointement une partie d'un système de noyau saturé ou insaturé, contenant facultativement jusqu'à deux hétéroatomes choisis entre N, O et S ;
dans la préparation d'un médicament pour le traitement d'un trouble prolifératif.

2. Utilisation suivant la revendication 1, dans laquelle R¹, R², R³ et R⁴ représentent chacun indépendamment H, un groupe NO₂, CF₃, SCF₃, CN, halogéno, OH, OR⁶, NH₂, NHR⁶, NR⁶R⁷, N⁺R⁶R⁷R⁸, COOH, COOR⁶, CONH₂, CONHR⁶, CONR⁶R⁷, COH, COR⁶, SR⁶, SOR⁶, SO₂R⁶, SO₂OH, SO₂OR⁶, SO₂NH₂, SO₂NHR⁶, SO₂R⁶R⁷, alkyle, cycloalkyle, cyclohétéroalkyle, aryle ou hétéroaryle.

3. Utilisation suivant la revendication 1 ou la revendication 2, dans laquelle chacun des groupes R⁶, R⁷ et R⁸ représente indépendamment un groupe hydrocarbyle, ou bien deux des groupes R⁶, R⁷ et R⁸ forment conjointement une partie d'un système de noyau saturé, contenant facultativement jusqu'à deux hétéroatomes choisis entre N, O et S.

4. Utilisation suivant l'une quelconque des revendications précédentes, dans laquelle chacun des groupes R⁹ et R¹⁰ représente indépendamment un groupe hydrocarbyle, ou bien R⁹ et R¹⁰ forment conjointement une partie d'un système de noyau saturé, contenant facultativement jusqu'à deux hétéroatomes choisis entre N, O et S.

5. Utilisation suivant l'une quelconque des revendications précédentes, dans laquelle le groupe hydrocarbyle est un groupe alkyle ou aryle.

6. Utilisation suivant l'une quelconque des revendications précédentes, dans laquelle R⁵ représente un groupe OH, CN, CONH₂, CONHNH₂, CONHOH ou CONR⁹R¹⁰.

7. Utilisation suivant la revendication 6, dans laquelle R⁹ et R¹⁰ forment conjointement une partie d'un système de noyau saturé.

8. Utilisation suivant l'une quelconque des revendications précédentes, dans laquelle R⁵ est choisi entre des groupes CONH₂, CO-pyrrolidine, CONHNH₂, CONHOH, CN ou OH.

9. Utilisation suivant l'une quelconque des revendications précédentes, dans laquelle R¹, R², R³ et R⁴ représentent chacun indépendamment H, un groupe NO₂, CF₃, SCF₃ ou halogène.

10. Utilisation suivant l'une quelconque des revendications précédentes, dans laquelle R² est choisi entre des groupes CF₃, F, SCF₃ et H .

11. Utilisation suivant l'une quelconque des revendications précédentes, dans laquelle R³ et R¹ représentent l'un et l'autre H.

12. Utilisation suivant l'une quelconque des revendications précédentes, dans laquelle R⁴ représente un groupe NO₂.

13. Utilisation suivant l'une quelconque des revendications précédentes, dans laquelle ledit composé de formule I est choisi entre les suivants :
amide d'acide 7-nitro-3-oxy-5-trifluorométhyl-benzothiazole-2-carboxylique ;
(7-nitro-3-oxy-5-trifluorométhyl-benzothiazole-2-yl)-pyrrolidine-1-yl-méthanone ;
hydrazide d'acide 7-nitro-3-oxy-5-trifluorométhyl-benzothiazole-2-carboxylique ;
amide d'acide 5-cyano-7-nitro-3-oxy-benzothiazole-2-carboxylique ;
hydroxamide d'acide 7-nitro-3-oxy-5-trifluorométhyl-benzothiazole-2-carboxylique ;
amide d'acide 5-fluoro-7-nitro-3-oxy-benzothiazole-2-carboxylique ;
hydroxamide d'acide 5-fluoro-7-nitro-3-oxy-benzothiazole-2-carboxylique ;
7-nitro-3-oxy-5-trifluorométhyl-benzothiazole-2-carbonitrile ;
7-nitro-3-oxy-5-trifluorométhyl-benzothiazole-2-ol ; et
amide d'acide 7-nitro-3-oxy-5-trifluorométhylsulfanyl-benzothiazole-2-carboxylique.

14. Utilisation suivant la revendication 13, dans laquelle le composé de formule I est choisi entre les suivants :
amide d'acide 7-nitro-3-oxy-5-trifluorométhyl-benzothiazole-2-carboxylique ;
amide d'acide 5-cyano-7-nitro-3-oxy-benzothiazole-2-carboxylique ;
7-nitro-3-oxy-5-trifluorométhyl-benzothiazole-2-carbonitrile ; et
amide d'acide 7-nitro-3-oxy-5-trifluorométhylsulfanyl-benzothiazole-2-carboxylique.

15. Utilisation suivant la revendication 13, dans laquelle le composé de formule I est choisi entre les suivants :
(7-nitro-3-oxy-5-trifluorométhyl-benzothiazole-2-yl)-pyrrolidine-1-yl-méthanone ;
amide d'acide 5-cyano-7-nitro-3-oxy-benzothiazole-2-carboxylique ;
amide d'acide 5-fluoro-7-nitro-3-oxy-benzothiazole-2-carboxylique ;
hydroxamide d'acide 5-fluoro-7-nitro-3-oxy-benzothiazole-2-carboxylique ;
7-nitro-3-oxy-5-trifluorométhyl-benzothiazole-2-carbonitrile ; et
amide d'acide 7-nitro-3-oxy-5-trifluorométhylsulfanyl-benzothiazole-2-carboxylique.

16. Utilisation suivant l'une quelconque des revendications précédentes, dans laquelle le trouble prolifératif est un cancer.

17. Utilisation suivant l'une quelconque des revendications 1 à 15, dans laquelle le trouble prolifératif est la glomérulonéphrite.

18. Utilisation suivant l'une quelconque des revendications 1 à 15, dans laquelle le trouble prolifératif est la polyarthrite rhumatoïde.

19. Utilisation suivant l'une quelconque des revendications 1 à 15, dans laquelle le trouble prolifératif est le psoriasis.

20. Utilisation suivant l'une quelconque des revendications 1 à 15, dans laquelle le trouble prolifératif est un trouble pulmonaire obstructif chronique.

21. Utilisation suivant l'une quelconque des revendications précédentes, dans laquelle ledit composé de formule I est administré en une quantité suffisante pour inhiber au moins une enzyme PLK.

22. Utilisation suivant la revendication 21, dans laquelle l'enzyme PLK est PLK1.

23. Utilisation suivant l'une quelconque des revendications précédentes, dans laquelle ledit composé de formule I est administré en association avec un excipient, diluant ou support pharmaceutiquement acceptable.

24. Utilisation suivant l'une quelconque des revendications précédentes, dans laquelle ledit composé de formule I est administré en association avec un agent antiprolifératif supplémentaire.

25. Composé choisi entre les suivants :
(7-nitro-3-oxy-5-trifluorométhyl-benzothiazole-2-yl)-pyrrolidine-1-yl-méthanone ;
amide d'acide 5-fluoro-7-nitro-3-oxy-benzothiazole-2-carboxylique ;
hydroxamide d'acide 5-fluoro-7-nitro-3-oxy-benzothiazole-2-carboxylique ; et
amide d'acide 7-nitro-3-oxy-5-trifluorométhylsulfanyl-benzothiazole-2-carboxylique.

26. Composition pharmaceutique comprenant un composé suivant la revendication 25, en mélange avec un diluant, excipient ou support pharmaceutiquement acceptable.

27. Utilisation d'un composé de formule I tel que défini dans l'une quelconque des revendications 1 à 15 dans une analyse pour identifier des composés candidats supplémentaires capables d'inhiber PLK.

28. Utilisation suivant la revendication 27, dans laquelle ladite analyse est une analyse de liaison compétitive.
